# EUROPEAN PATENT SPECIFICATION

(11) **EP 1 496 793 B1**
(45) Date of publication and mention of the grant of the patent: **09.08.2006**
(21) Application number: 03720724.8
(22) Date of filing: 22.04.2003
(51) Int. Cl.: A61B 3/16, A61B 8/06

(54) **VASCULAR IMPEDANCE MEASUREMENT APPARATUS AND METHOD**
VORRICHTUNG UND VERFAHREN ZUR MESSUNG DER VASKULÄREN IMPEDANZ
APPAREIL ET PROCÉDÉ DE MESURE DE L'IMPEDANCE VASCULAIRE

(30) Priority: 19.04.2002 GB 0208945
(43) Date of publication of application: 19.01.2005
(73) Proprietor: The Queen's University of Belfast, Belfast BT7 1NN (GB)
(72) Inventor: MCVEIGH, Gary, Eugene, Belfast BT9 6FX (GB)
(74) Representative: Cooper, John
(86) International application number: PCT/GB2003/001729
(87) International publication number: WO 2003/088829

(56) References cited:
- POLSKA E ET AL: "RI in central retinal artery as assessed by CDI does not correspond to retinal vascular resistance." AMERICAN JOURNAL OF PHYSIOLOGY. HEART AND CIRCULATORY PHYSIOLOGY. UNITED STATES APR 2001, vol. 280, no. 4, April 2001 (2001-04), pages H1442-H1447, XP002254524 ISSN: 0363-6135
- POWALOWSKI T ET AL: "NON-INVASIVE ULTRASONIC METHOD FOR THE BLOOD FLOW AND PRESSURE MEASUREMENTS TO EVALUATE THE HEMODYNAMIC PROPERTIES OF THE CEREBRO-VASCULAR SYSTEM" ARCHIVES OF ACOUSTICS, POLISH SCIENTIFIC PUBLISHERS, WARZAW, PL, vol. 10, no. 3, 1985, pages 303-314, XP000646828 ISSN: 0137-5075

## Description

The present invention relates to apparatus for measuring vascular impedance.

The complications of cardiovascular disease represent the leading cause of morbid and mortal events in Western society. At present, diagnostic procedures are designed to assess the extent and severity of blood vessel damage when symptoms present or with the occurrence of vascular events. The diagnostic challenge is to detect abnormal structure and function in the vascular system at an early pre-clinical stage. The ability to detect and monitor sub-clinical arterial damage has the potential to refine cardiovascular risk stratification and enable early intervention to prevent or attenuate disease progression.

Traditionally, the arterial circulation has been considered a steady-flow system characterised by mean arterial pressure that represents the product of cardiac output and total peripheral resistance.

The pulsatile component of pressure is determined by the pattern of left ventricular ejection and the stroke volume. The compliance characteristics of the arterial circulation has been largely ignored in prior haemodynamic studies.

The importance of assessing arterial wall integrity has been highlighted by studies demonstrating that a reduction in the pulsatile function or compliance characteristics of large arteries represents a powerful independent risk factor for future cardiovascular events. Accumulating evidence suggests that abnormalities in the pulsatile characteristics of arteries occur early in disease processes associated with increased cardiovascular risk. Importantly, impaired pulsatile arterial function is recognised as an independent predictor of risk for vascular events in patients with various disease states including coronary heart disease, congestive heart failure, hypertension and diabetes mellitus.

Studies relating outcome to abnormalities in pulsatile function have focused on large arteries, although analysis of arterial pressure pulse waveforms suggest that the earliest abnormalities in arterial structure and function resides in the microcirculation.

The study of this section of the vasculature has been hindered by the lack of a non-invasive, reproducible and repeatable technique capable of assessing the compliance characteristics or pulsatile function of small arteries and arterioles.

Physiologically, the impedance load or opposition to flow presented by the circulation is measured invasively by analysing the altered pressure/flow relationships and pulse contour parameters produced through the effects of disease on the structural and functional components of the arterial system. Input impedance relates simultaneously recorded pressure and flow waveforms under specific mathematical conditions. The haemodynamic properties of the system can be quantified as the impedance concept permits the heart and arteries to be considered separately and their interaction understood as a function of pump and load properties. As pressure and flow waves are periodic and continuous, Fourier series methods can be used to generate the impedance function. The modulus at each harmonic in the Fourier series is the ratio of the pressure modulus to the flow modulus at that harmonic and the phase at each harmonic is the difference between pressure phase and flow phase at the same harmonic. As the impedance of a vascular bed varies with frequency, complete specification of pulsatile pressure and flow relationships takes the form of the spectrum of moduli and phase angles versus frequency⁵.

Characteristic impedance (the inverse of arterial compliance) defines the relationship between pressure and flow in an artery or arterial network when pressure and flow waves are not influenced by wave reflections. These conditions do not exist in the arterial system and the input impedance values oscillate around the characteristic impedance value because of wave reflection. Wave reflections are known to exert their greatest influence on impedance moduli at low frequencies. For higher frequencies, the input impedance approaches the characteristic impedance which has been estimated in prior haemodynamic studies as the arithmetic mean of input impedance moduli above 2-4 Hz.

In the prior art, detailed studies of arterial pressure and flow are only possible through the use of invasive techniques. Such techniques cannot be used to monitor changes in the circulatory system of a patient over time because of the dangers to health posed by these techniques.

In accordance with a first aspect of the present invention there is provided apparatus for the measurement of vascular impedance of the ocular micro circulation *in vivo,* the apparatus comprising intra-ocular pressure measurement means, from which a pressure pulse waveform is calculable and blood velocity profile measurement means for measuring the linear blood flow velocity waveform in the retrobulbar circulation, means for calculating a vascular impedance modulus from the pressure pulse waveform and the linear blood flow velocity, as a function of frequency.

Preferably the intra-ocular pressure measurement means is suitable for measuring the maximum and minimum pressure values of the pulse profile to calculate a mean intra-ocular pressure.

Preferably, the apparatus is suitable for measuring how the pressure pulse waveform and the linear blood flow velocity vary over the period of a respiratory cycle.

Preferably, the means for calculating the vascular impedance modulus takes into account the

Preferably, a solid state transducer is used to measure intra-ocular pressure.

Preferably, the solid state transducer operates in conjunction with a suitable telemetry system to process the data.

Optionally, an ocular pneumotonometer is used to measure intra-ocular pressure.

Preferably the blood velocity profile measurement means is an ultrasound device.

Preferably the ultrasound device is a doppler ultrasound imager.

Preferably, the apparatus further comprises motion picture generation means to produce moving images of an artery.

Preferably, the moving images are capable of being used to ensure that a user of the apparatus can accurately identify the location of an artery.

Preferably the change in the pulsatile intra-ocular pressure waveform and the linear blood flow velocity are measured sequentially.

Preferably, the means for calculating the vascular impedance modulus comprises obtaining the fourier transform of the intra-ocular pressure pulse waveform and the linear blood flow velocity and dividing the transformed values of the pulsatile change in the intra-ocular pressure pulse by the transformed retrobulbar blood flow velocity.

Preferably the pulsatile change in intra-ocular pressure has a phase associated therewith.

Preferably the intra-ocular blood velocity has a phase associated therewith.

In accordance with a second aspect of the present invention there is provided a method for the measurement of vascular impedance of the ocular micro circulation *in vivo*, the method comprising the steps of: measuring the intra-ocular pressure pulse waveform of the ocular network;
measuring the linear blood flow velocity wavefrom in the retrobulbar circulation; and
calculating a vascular impedance modulus from the intra ocular pressure pulse waveform and the linear blood flow velocity waveform, as a function of frequency.

Preferably, the pressure pulse waveform and the linear blood flow velocity are measured over the period of a respiratory cycle, and their variation therewith is measured.

Preferably, the variations are used in the calculation of the vascular impedance modulus.

Preferably, the method further comprises the steps of recording moving images of an artery.

Preferably, the moving images are used to accurately identify the location of an artery.

Preferably, the change in the pulsatile intra-ocular pressure waveform and the linear blood flow velocity are measured sequentially.

Preferably, the step of calculating the vascular impedance modulus comprises the steps of; obtaining the fourier transform of the intra-ocular pressure pulse waveform and the linear blood flow velocity and dividing the transformed values of the pulsatile change in the intra-ocular pressure pulse by the transformed retrobulbar blood flow velocity.

The invention will now be described by way of example only with reference to the accompanying drawings in which:
Fig. 1 is a diagram of an eye having means for measuring the intra-ocular pressure using the principle of applanation tonometry at the front of the eye;
Fig. 2 is a diagram of an eye having means for measuring the linear flow velocity by interrogating the retrobulbar circulation from the front of the eye;
Fig. 3 is a graph of the periodic pressure signal as measured using the present invention plotted against time;
Fig. 4 is a graph of the periodic velocity signal as measured using the present invention plotted against time;
Fig. 5 is a graph of impedance modulus plotted against frequency; and
Fig. 6 is a graph of phase plotted against frequency.

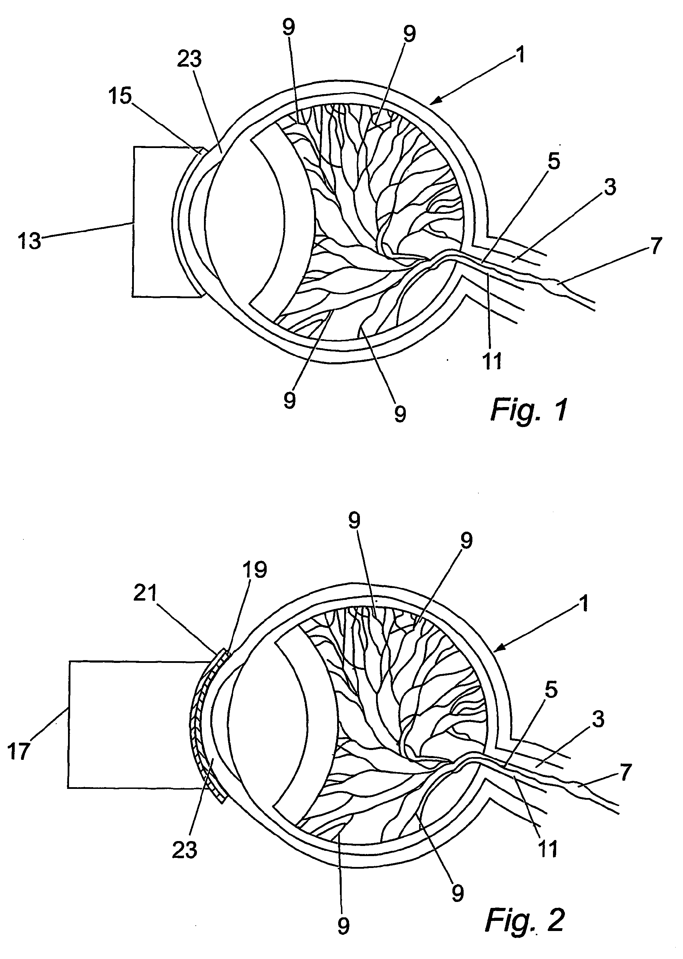

Figs. 1 and 2 show a first embodiment of the present invention. Figs. 1 and 2 are diagrams showing some features of the human eye 1. These include the optic nerve 3, the ophthalmic artery 5, a bolus of blood contained in the ophthalmic artery 5 positioned outside the ocular vascular network 9. The vein 11 is also shown.

Fig. 1 also shows the means for measuring the intra-ocular pressure 13, provided, in this example by a tonometer system applanated to the cornea 23.

Fig. 2 shows means for measuring the linear blood flow velocity in the retrobulbar circulation 17, connected to the front of the eye. This is an ultrasonic device that is placed on the eyelid 19,the eyelid 19 being covered with a gel 21 to ensure that the ultrasound device is properly coupled to the eye 1. This device measures the linear velocity of the bolus of blood 7 in the ophthalmic artery 5.

The tonometer system 13 used can employ continuous airflow pneumotonometry (for example using an airflow pneumotonometer as provided by Paradigm Medical Industries) or can use a solid state transducer (for example as supplied by Smart Lens DCT) together with suitable telemetry system to process the detected data. The arterial function has been found to have a significant dynamic range of approximately 0-12 Hz, and thus, the choice of a pneumatic versus a solid state transducer system will depend on a suitable dynamic range being provided by the particular tonometer device used. A probe 15 is applanated on the cornea 23 to record intraocular pressure. The tonometer device 13 samples at 200 Hz with a resolution of 0.01 mmHg and the signals are acquired over a 20 second period. Pulsatile variation of intraocular pressure results from pressure oscillations generated by cardiac contraction altering the distending pressure in the vessel walls. Compliance of an artery, or an entire arterial bed, describes the ability to store a varying amount of blood. Changes in volume within the ocular vascular bed will produce an equal change in volume. The pulsatile ocular waveforms are recorded after administration of oxybuprocaine 0.4% drops to anaesthetise the cornea.

The variation in intra-ocular pressure as a function of time reflects the introduction of the bolus of blood 7 into the ocular vascular network 9. The ocular vascular network 9 expands to accommodate the additional volume of blood.

As the intra-ocular fluids are incompressible, the intra-ocular pressure response to the volume change will depend of the viscoelastic properties of the vessel network and the ocular rigidity. The mechanical properties and distending pressures will vary at different sites in the ocular vascular network 9 and it is the composite effect of these influences that determine the intra-ocular pressure waveform morphology. Whilst the rigidity of the ocular coat can vary between individuals, the half-life of the collagen and elastin components are measured in years. Consequently, the characteristics of these boundary structures would not be expected to change significantly within an individual over a period of weeks or months. Therefore changes recorded in the intra-ocular pressure pulse waveform will be reflective of alteration in the viscoelastic properties of the ocular microcirculatory bed.

The present invention uses the directly recorded change in intra-ocular pressure in its analysis and not the generated flow output measurements from the device that relate pressure change to volume change within the eye. The pulsatility of the intra-ocular pressure is dependent on the pulsatile inflow and distension of the vessels which is related to the viscoelastic properties of the ocular circulation. Scleral rigidity may limit the frequency of pressure fluctuations but does not cause variation in pressure.

In the example shown in Fig. 2, a colour doppler ultrasound imager 17 is used to examine the blood velocity waveform in the retrobulbar ocular circulation. The ultrasound imager may suitably be a Phillips ATL HDI3500 Ultrasound Machine.

The appropriate blood vessels then have to be located and identified. One way of doing this is to employ simultaneous B-scan and doppler imaging. However, there are a number of practical difficulties that have to be overcome when performing this. Firstly, the orbit is three dimensional but viewing is possible only in two dimensions using the ultrasound machine. Furthermore, the ophthalmic artery is tortuous and has many branches and so it is difficult to get clear views and for the operator to know exactly where he is looking. There are also wide anatomical variations in the position and branching nature of the ophthalmic artery between individuals.

These problems have been addressed by recording real-time colour motion pictures when initially inspecting the artery in a subject. They are then played back under 'cineloop review' and, in conjunction with depth measurements, used to orientated the operator back to the original recording site. Pre-recorded velocity waveforms finally verify dimensional and morphological authenticity of waveforms under view.

The beam from the ultrasound imager can be focussed using an appropriate software algorithm.

The sample volume defined by the imager 17 is placed over a vessel of interest, in this case, the bolus of blood 7 and the frequency shifts received are assembled into a spectral waveform. The spectral waveform represents the cumulative frequency shifts present and can be displayed as a time-velocity waveform.

In use, alternate measurements of the arterial pulse waveform and blood velocity profile are taken.

The shape of the linear velocity flow waveform, recorded in the retrobulbar circulation , is determined by and is critically dependent on changes in total cross-sectional area of the ocular vascular network.

Like pressure, flow will also vary at different sites in the ocular vascular network 9 and the velocity waveform morphology therefore reflects the status of the entire ocular vascular network 9. In essence, the flow velocity waveform derived from the retrobulbar circulation and the intra-ocular pressure waveform reflect the sum total of the various calibre and pressure changes throughout the ocular vascular bed.

Measured over time, changes in the linear flow waveform can provide information on changes in the ability of the ocular vascular network to expand during the cardiac cycle. Such information can lead to early diagnosis and subsequent early treatment of disease.

The present invention uses linear velocity of flow in calculating the vascular impedance of the microcirculation as changes in velocity of flow are determined by changes in the total cross-sectional area of the ocular vascular network 9. Furthermore, the use of linear velocity of flow permits comparisons of impedance moduli derived from different arteries and in the same artery under varying conditions. This comparison cannot be validly made using volume flow measurements.

Previous work to characterise the arterial system has been based on the relationship between pressure and flow recorded at the same position in time and space. Windkessel analysis is used to apply an electrical circuit analogy of input impedance to fit components of total compliance and total resistance to the distal arterial tree. However, this technique does not provide unique solutions.

In contrast to previous work, the present invention provides for the recording of pressure and velocity waveforms at different positions on the arterial tree. In the ocular microcirculation, ophthalmic flow can be considered giving rise to the intraocular pressure. This means that an analogy can be drawn with two port analysis of electrical circuit design, which relates an input signal to an output signal. The relationship between the intraocular pressure and the corresponding ophthalmic velocity waveform can thus be characterised.

The waveforms of pressure and velocity have a certain periodicity according to the heart rate of the subject being tested. However, the breathing of the subject also affects the waveforms. Hence, a measure of compliance can be made that takes into account the respiratory variations. This overcomes an assumption made by use of a normal Windkessel analysis, namely that the pressure flow waveform has an infinite pulse wave velocity. This measure of compliance that takes the respiratory variations into account can be known as the apparent compliance. It can be used in conjunction with the two port model to characterise the system.

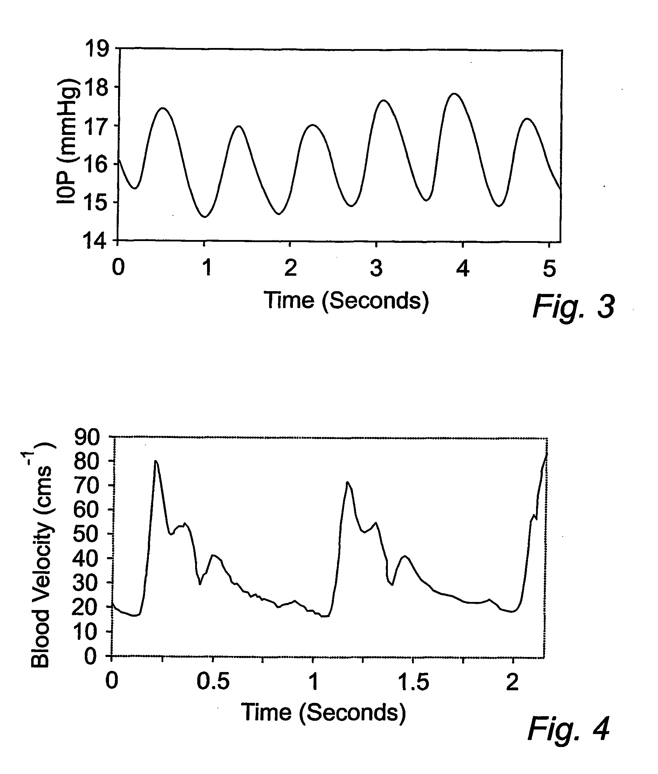

Typical examples of intraocular pressure and velocity profiles (obtained from the ophthalmic artery) are shown in Figures 3 and 4.

Fig. 3 is a graph of pressure plotted with respect to time. The figure shows the periodicity of the pressure fluctuation. The cardiac cycle can be identified from the period of the pressure fluctuation as being approximately 0.9 s.

Fig.4 is a graph of linear blood velocity plotted with respect to time. The figure shows the periodicity of linear velocity fluctuation. The cardiac cycle can be identified from the period of the linear velocity fluctuation as being approximately 0.9s.

The sites of data acquisition enable the recording of pressure and linear velocity waveforms that provide information about the entire ocular vascular network and not merely single vessel in the network. Measurements are obtained sequentially using the tangent method to align pressure and velocity waveforms. This technique is employed to ensure effective alignment of waveforms for analysis. The signals may also be gated to an ECG. Other known methods may also be employed.

As seen in Figures 3 and 4, the velocity and pressure signals are periodic and time dependent and can thus be represented in the frequency domain by obtaining their Fourier transform: P(ω) = FT [P(t)] and V (ω) = FT[V(t)] where FT represents Fourier transformation. In addition, each frequency component of pressure and velocity will have its own associated phase (Øp pressure phase, Øv velocity phase). The frequency dependent impedance modulus and phase can be determined from: Z (ω) = P (ω) /V (ω) and Ø (ω) = Øp(ω) - ØV(ω) .

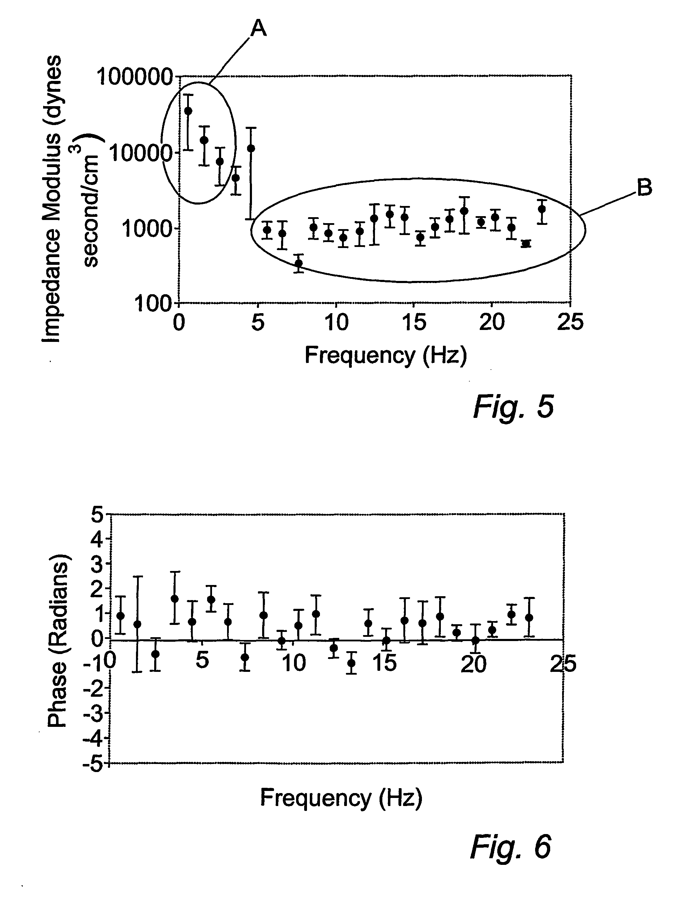

Figures 5 and 6 show typical plots of Z (ω) and Ø(ω) for a normal subject.

The flow and first derivative of pressure occur at similar time points. As pressure and flow are obtained sequentially the first derivative of the pressure waveform is aligned to the flow waveform. A tangent to end diastole and a tangent to the initial upstroke in pressure wall intersect at the "foot" of the waveform. This point is aligned with the same point on the flow waveform.

An improved alignment can be obtained by synching the peak velocity detected by the imager 17 to an ECG device.

Frequency domain analysis provides information about steady-state (resistance) and pulsatile function (characteristic impedance) of the ocular circulation. In Fig. 5, the steady state resistance is shown in area A and the characteristic impedance in area B. These signals are stored in digital form and the digitised signals are amenable to analysis in the time domain with the application of mathematical models to interpret waveshape changes in relation to the mechanical properties of the ocular circulatory bed.

The present invention is highly advantageous with respect to the prior art because it provides a non-invasive method and apparatus for measuring vascular impedance and in particular, through interrogation of the wave shape, of the linear velocity profile of the blood bolus in the retrobulbar circulation. Previously, invasive techniques had only been thought capable of providing information on the linear velocity profile. Such techniques are expensive and cannot be used to obtain repeat results over a period of time for the same subject. The present invention therefore allows a physician to monitor changes in the microcirculation of the eye and to extrapolate the data to make clinical judgements in various disease states associated with an increase in cardiovascular events.

The present invention is applicable in a number of areas of clinical research. Some examples are given below.

It has been recognised for many years that characteristic changes in the arterial pressure pulse contour occur in many disease states and with physiological and pharmacological interventions. Alteration in arterial waveform morphology typically involves a steepening of the diastolic decay and a diminution in the amplitude and duration of the oscillatory waveform that distorts the proximal part of diastole from a pure monoexponential. The oscillatory diastolic waveform arises from wave reflection and damped resonance occurring in the arterial tree with the major sites of reflected waves originating in smaller arteries and arterioles. Loss of the oscillatory diastolic waveform is recognised as an early marker of altered vessel wall properties that identifies impaired pulsatile function of arteries as it can be found in patients at increased cardiovascular risk without alteration in total peripheral resistance. This has been demonstrated in patients with diabetes mellitus and cigarette smokers. Whilst the microvascular changes associated with diabetes are well recognised, the structural changes that are commonly found in the arterioles of smokers and rarely in non-smokers, are less well appreciated. These microvascular abnormalities may account for the common occurrence of microinfarcts found in association with diabetes and cigarette smoking that have hitherto gone unrecognised.

Analysis of the arterial pressure pulse waveform can also be useful in identifying the haemodynamic action of drug therapy not detected by the traditional measurement of peripheral resistance.

Improvements and modifications may be incorporated herein without deviating from the scope of the invention, which is defined in the claims.

## Claims

1. Apparatus for the measurement of vascular impedance of the ocular micro circulation *in vivo,* comprising intra-ocular pressure measurement means (13) from which a pressure pulse waveform is calculable, blood velocity profile measurement means (17) for measuring the linear blood flow velocity waveform in the retrobulbar circulation, and means for calculating a vascular impedance modulus from the pressure pulse waveform and the linear blood flow velocity waveform, as a function of frequency.

2. Apparatus as claimed in claim 1, wherein the intra-ocular pressure measurement means (13) is suitable for measuring the maximum and minimum pressure values of the pulse profile to calculate a mean intra-ocular pressure.

3. Apparatus as claimed in claim 1 or claim 2, suitable for measuring how the pressure pulse waveform and the linear blood flow velocity vary over the period of a respiratory cycle.

4. Apparatus as claimed in any preceding claim, wherein a solid state transducer is used to measure intra-ocular pressure.

5. Apparatus as claimed in claim 4, wherein a suitable solid state transducer operates in conjunction with a suitable telemetry system to process the data.

6. Apparatus as claimed in any of claims 1 to 3, wherein an ocular pneumotonometer is used to measure intra-ocular pressure.

7. Apparatus as claimed in any preceding claim, wherein the blood velocity profile measurement means (17) is an ultrasound device.

8. Apparatus as claimed in claim 7, wherein the ultrasound device is a doppler ultrasound imager.

9. Apparatus as claimed in any preceding claim further comprising motion picture generation means to produce moving images of an artery.

10. Apparatus as claimed in claim 9, wherein the moving images are capable of being used to ensure that a user of the apparatus can accurately identify the location of an artery.

11. Apparatus as claimed in any preceding claim, wherein the means for calculating the vascular impedance modulus comprises means for;
obtaining the fourier transform of the intra-ocular pressure pulse waveform and the linear blood flow velocity and dividing the transformed values of the pulsatile change in the intra-ocular pressure pulse by the transformed retrobulbar blood flow velocity.

12. A method for the measurement of vascular impedance of the ocular micro circulation *in vivo,* comprising the steps of: measuring the intra-ocular pressure pulse waveform of the ocular network; measuring the linear blood flow velocity waveform in the retrobulbar circulation; and
calculating the vascular impedance modulus from the intra ocular pressure pulse waveform and the linear blood flow velocity waveform, as a function of frequency.

13. A method as claimed in claim 12, wherein the pressure pulse waveform and the linear blood flow velocity are measured over the period of a respiratory cycle, and their variation therewith is measured.

14. A method as claimed in claim 13, wherein the variations are used in the calculation of the vascular impedance modulus.

15. A method as claimed in any of claims 12 to 14, further comprising the steps of recording moving images of an artery.

16. A method as claimed in claim 15, wherein the moving images are used to accurately identify the location of an artery.

17. A method as claimed in any of claims 12 to 16, wherein the change in the pulsatile intra-ocular pressure waveform and the linear blood flow velocity are measured sequentially.

18. A method as claimed in any of claims 12 to 17, wherein the step of calculating the vascular impedance modulus comprises the steps of;
obtaining the fourier transform of the intra-ocular pressure pulse waveform and the linear blood flow velocity and dividing the transformed values of the pulsatile change in the intra-ocular pressure pulse by the transformed retrobulbar blood flow velocity.

## Patentansprüche

1. Eine Vorrichtung zur Messung vaskulärer Impedanz der okularen Mikrozirkulation *in vivo,* die ein intra-okulares Druckmessmlttel (13), mit dem eine Druckpulswellenform kalkulierbar ist, ein Blutgeschwindigkeitsprofilmessmittel (17) zum Messen der Wellenform der linearen Blutflussgeschwindigkeit in der retrobulbären Zirkulation und ein Mittel zum Kalkulieren eines vaskulären Impedanzmoduls aus der Wellenform der Druckpulswellenform und der Wellenform der linearen Blutflussgeschwindigkeit als eine Frequenzfunktion, umfasst.

2. Vorrichtung gemäß Anspruch 1, wobei das intra-okulare Druckmessmittel (13) zum Messen des maximalen und minimalen Druckwerts des Pulsprofils zum Kalkulieren eines mittleren intra-okularen Drucks geeignet ist.

3. Vorrichtung gemäß Anspruch 1 oder Anspruch 2, die zum Messen, wie sich die Druckpulswellenform und die lineare Blutflussgeschwindigkeit über die Zeitspanne eines Atmungszyklus ändern, geeignet ist.

4. Vorrichtung gemäß einem der vorhergehenden Ansprüche, wobei zum Messen des intra-okularen Drucks ein Festkörperwandler verwendet wird.

5. Vorrichtung gemäß Anspruch 4, wobei zum Verarbeiten der Daten ein geeigneter Festkörperwandler in Verbindung mit einem geeigneten Telemetriesystem funktioniert.

6. Vorrichtung gemäß einem der Ansprüche 1 bis 3, wobei zum Messen des intra-okularen Drucks ein okularer Pneumotonometer verwendet wird.

7. Vorrichtung gemäß einem der vorhergehenden Ansprüche, wobei das Blutgeschwindigkeitsprofilmessmittel (17) ein Ultraschallgerät ist.

8. Vorrichtung gemäß Anspruch 7, wobei das Ultraschallgerät ein Doppler-Ultraschall-Bildgenerator ist.

9. Vorrichtung gemäß einem der vorhergehenden Ansprüche, die ferner ein Filmerzeugungsmittel zum Produzieren von Bewegtbildern einer Arterie beinhaltet.

10. Vorrichtung gemäß Anspruch 9, wobei die Bewegtbilder verwendet werden können, um sicherzustellen, dass ein Benutzer der Vorrichtung die Lage einer Arterie genau bestimmen kann.

11. Vorrichtung gemäß einem der vorhergehenden Ansprüche, wobei das Mittel zum Kalkulieren des vaskulären Impedanzmoduls Mittel für Folgendes beinhaltet:
Erhalten der Fourier-Transformation der intra-okularen Druckpulswellenform und der linearen Blutflussgeschwindigkeit und Unterteilen der transformierten Werte der pulsatilen Veränderung des intra-okularen Druckpulses durch die transformierte retrobulbäre Blutflussgeschwindigkeit.

12. Ein Verfahren zur Messung vaskulärer Impedanz der okularen Mikrozirkulation *in vivo,* das folgende Schritte beinhaltet: Messen der intra-okularen Druckpulswellenform des okularen Netzes; Messen der Wellenform der linearen Blutflussgeschwindigkeit in der retrobulbären Zirkulation; und Kalkulieren des vaskulären Impedanzmoduls aus der intra-okularen Druckpulswellenform und der Wellenform der linearen Blutflussgeschwindigkeit als eine Frequenzfunktion.

13. Verfahren gemäß Anspruch 12, wobei die Druckpulswellenform und die lineare Blutflussgeschwindlgkeit über die Zeitspanne eines Atmungszyklus gemessen werden und ihre Änderung damit gemessen wird.

14. Verfahren gemäß Anspruch 13, wobei die Änderungen bei der Kalkulation des vaskulären Impedanzmoduls verwendet werden.

15. Verfahren gemäß einem der Ansprüche 12 bis 14, das ferner die Schritte des Aufnehmens von Bewegtbildern einer Arterie beinhaltet.

16. Verfahren gemäß Anspruch 15, wobei die Bewegtbilder zur genauen Bestimmung der Lage einer Arterie verwendet werden.

17. Verfahren gemäß einem der Ansprüche 12 bis 16, wobei die Veränderung der pulsatilen intra-okularen Druckpulswellenform und der linearen Blutflussgeschwindigkeit aufeinanderfolgend gemessen werden.

18. Verfahren gemäß einem der Ansprüche 12 bis 17, wobei der Schritt des Kalkulierens des vaskulären Impedanzmoduls folgende Schritte beinhaltet: Erhalten der Fourier-Transformation der intra-okularen Druckpulswellenform und der linearen Blutflussgeschwindigkeit und Unterteilen der transformierten Werte der pulsatilen Veränderung des intra-okularen Druckpulses durch die transformierte retrobulbäre Blutflussgeschwindigkeit.

## Revendications

1. Appareil destiné à la mesure de l'impédance vasculaire de la microcirculation oculaire in vivo, comportant un moyen de mesure de la pression intra-oculaire (13) d'après lequel une forme d'onde de l'impulsion de pression peut être calculée, un moyen de mesure du profil du débit sanguin (17) destiné à mesurer la forme d'onde du débit sanguin linéaire dans la circulation rétrobulbaire, et un moyen destiné à calculer un module d'impédance vasculaire d'après la forme d'onde de l'impulsion de pression et la forme d'onde du débit sanguin linéaire, en fonction de la fréquence.

2. Appareil tel que revendiqué dans la revendication 1, dans lequel le moyen de mesure de la pression intra-oculaire (13) convient à la mesure des valeurs de pression maximum et minimum du profil d'impulsion pour calculer une pression intra-oculaire moyenne.

3. Appareil tel que revendiqué dans la revendication 1 ou la revendication 2, convenant pour mesurer la façon dont la forme d'onde de l'impulsion de pression et le débit sanguin linéaire varient au cours de la période d'un cycle respiratoire.

4. Appareil tel que revendiqué dans n'importe quelle revendication précédente, dans lequel un transducteur à état solide est utilisé pour mesurer la pression intra-oculaire.

5. Appareil tel que revendiqué dans la revendication 4, dans lequel un transducteur à état solide approprié fonctionne en conjonction avec un système de télémétrie approprié pour traiter les données.

6. Appareil tel que revendiqué dans n'importe lesquelles des revendications 1 à 3, dans lequel un pneumotonomètre oculaire est utilisé pour mesurer la pression intra-oculaire.

7. Appareil tel que revendiqué dans n'importe quelle revendication précédente, dans lequel le moyen de mesure du profil du débit sanguin (17) est un dispositif à ultrasons.

8. Appareil tel que revendiqué dans la revendication 7, dans lequel le dispositif à ultrasons est un imageur à ultrasons à effet doppler.

9. Appareil tel que revendiqué dans n'importe quelle revendication précédente comportant de plus un moyen de génération d'images animées pour produire des images animées d'une artère.

10. Appareil tel que revendiqué dans la revendication 9, dans lequel les images animées sont à même d'être utilisées pour faire en sorte qu'un utilisateur de l'appareil puisse identifier avec précision l'emplacement d'une artère.

11. Appareil tel que revendiqué dans n'importe quelle revendication précédente, dans lequel le moyen destiné à calculer le module de l'impédance vasculaire comporte un moyen pour :
obtenir la transformée de Fourier de la forme d'onde de l'impulsion de pression intra-oculaire et du débit sanguin linéaire et diviser les valeurs transformées du changement pulsatile dans l'impulsion de pression intra-oculaire par le débit sanguin rétrobulbaire transformé.

12. Une méthode destinée à la mesure de l'impédance vasculaire de la microcirculation oculaire in vivo, comportant les étapes : de mesurer la forme d'onde de l'impulsion de pression intra-oculaire du réseau oculaire ; de mesurer la forme d'onde du débit sanguin linéaire dans la circulation rétrobulbaire ; et de calculer le module de l'impédance vasculaire à partir de la forme d'onde de l'impulsion de pression intra-oculaire et la forme d'onde du débit sanguin linéaire, en fonction de la fréquence.

13. Une méthode telle que revendiquée dans la revendication 12, dans laquelle la forme d'onde de l'impulsion de pression et le débit sanguin linéaire sont mesurés au cours de la période d'un cycle respiratoire, et leur variation avec celui-ci est mesurée.

14. Une méthode telle que revendiquée dans la revendication 13, dans laquelle les variations sont utilisées dans le calcul du module de l'impédance vasculaire.

15. Une méthode telle que revendiquée dans n'importe lesquelles des revendications 12 à 14, comportant de plus les étapes d'enregistrer des images animées d'une artère.

16. Une méthode telle que revendiquée dans la revendication 15, dans laquelle les images animées sont utilisées pour identifier avec précision l'emplacement d'une artère.

17. Une méthode telle que revendiquée dans n'importe lesquelles des revendications 12 à 16, dans laquelle les changements dans la forme d'onde de la pression intra-oculaire pulsatile et le débit sanguin linéaire sont mesurés de façon séquentielle.

18. Une méthode telle que revendiquée dans n'importe lesquelles des revendications 12 à 17, dans laquelle l'étape de calculer le module d'impédance vasculaire comporte les étapes : d'obtenir la transformée de Fourier de la forme d'onde de l'impulsion de pression intra-oculaire et du débit sanguin linéaire et de diviser les valeurs transformées du changement pulsatlle dans l'impulsion de pression intra-oculaire par le débit sanguin rétrobulbaire transformé.
